# EUROPEAN PATENT SPECIFICATION

(11) **EP 3 034 678 B1**
(45) Date of publication and mention of the grant of the patent: **05.07.2017**
(21) Application number: 15201275.3
(22) Date of filing: 18.12.2015
(51) Int. Cl.: D06F 58/20, D06F 73/02, D06F 58/10

(54) **CLOTHES TREATMENT APPARATUS INCLUDING A SCENT DIFFUSER**
VORRICHTUNG ZUR BEHANDLUNG VON KLEIDUNGSSTÜCKEN MIT EINEM DUFTDIFFUSOR
APPAREIL DE TRAITEMENT DE LINGE COMPRENANT UN DIFFUSEUR DE PARFUM

(30) Priority: 19.12.2014 KR 20140184786
(43) Date of publication of application: 22.06.2016
(73) Proprietor: LG Electronics Inc., Seoul 07336 (KR)
(72) Inventor: CHOI, Junyoung, 08592 Seoul (KR); DOH, Youngjin, 08592 Seoul (KR); NAM, Wansik, 08592 Seoul (KR)
(74) Representative: Vossius & Partner Patentanwälte Rechtsanwälte mbB

(56) References cited:
- EP-A1- 0 271 453
- EP-A1- 2 431 516
- EP-A1- 2 657 391
- WO-A2-2011/126318
- CN-Y- 2 745 983
- CN-Y- 201 172 761
- DE-B3- 10 318 486
- DE-U1- 7 935 996
- KR-A- 20110 043 250
- KR-A- 20140 016 093
- KR-Y1- 200 472 951
- US-A1- 2005 050 762
- US-A1- 2009 307 924
- US-A1- 2010 011 606

## Description

The present invention relates to a scent diffuser and a clothes treatment apparatus including the same.

Clothes treatment apparatuses are apparatuses that treat clothes, e.g. wash and dry clothes and smooth wrinkles in clothes, at home or at laundromats.

Clothes treatment apparatuses may be classified into a washer for washing clothes, a dryer for drying clothes, a washer/dryer having both a washing function and a drying function, a refresher for refreshing clothes, and a steamer for removing unnecessary wrinkles in clothes.

The refresher is an apparatus that keeps clothes comfortable and fresh. The refresher functions to dry clothes, to supply fragrance to clothes, to prevent the occurrence of static electricity in clothes, or to remove wrinkles from clothes.

The steamer is an apparatus that simply supplies steam to clothes in order to remove wrinkles from the clothes. Unlike a general iron, the steamer removes wrinkles from the clothes without directly applying heat to the clothes.

A clothes treatment apparatus having both functions of a refresher and a steamer may remove wrinkles from clothes received in the clothes treatment apparatus, and may additionally deodorize the clothes, using steam and hot air.

A scent diffuser configured to diffuse scent into a treatment chamber, in which clothes are received, may be used in a clothes treatment apparatus.

However, conventional scent diffusers have problems in that it is not possible for users to arbitrarily adjust the amount or concentration of scent to be discharged.

A clothes treatment apparatus comprising a scent diffuser according to the preamble of the independent claim 1 is disclosed by KR 2011 0043250 A. Other prior art laundry treatment apparatuses with scent diffusers are disclosed by KR 200 472 951 Y1, CN 201 172 761 Y, EP 2 431 516 A1, DE 103 18 486 B3, WO 2011/126318 A2, US 2010/011606 A1 and EP 2 657 391 A1. Therefore, the present invention has been made in view of the above problems.

It is an object of the present invention to provide a scent diffuser that is capable of enabling a user to arbitrarily adjust the amount or concentration of scent to be discharged and a clothes treatment apparatus including the same.

It is another object of the present invention to provide a scent diffuser configured to have a structure in which the amount or concentration of scent to be discharged is adjusted based on a user's manipulation without using additional electric power and a clothes treatment apparatus including the same.

It is another object of the present invention to provide a scent diffuser configured such that a discharge port can be used as a movement path and a clothes treatment apparatus including the same.

It is a further object of the present invention to provide a scent diffuser configured such that a user can intuitively check the extent of opening of a discharge port and a clothes treatment apparatus including the same.

In accordance with the present invention, the above and other objects can be accomplished by the provision of a laundry treatment apparatus comprising a scent diffuser according to the features of the independent claim 1. The opening adjustment port may include a plurality of opening adjustment ports, and the opening adjustment ports and the manipulation part may be arranged in a line.

The slider may be provided with an interference protrusion, the holder may include at least one positioning protrusion configured to interfere with the interference protrusion when the slider is moved, and the overlapping area between the discharge port and the opening adjustment port may be maintained by interference between the positioning protrusion and the interference protrusion.

The holder includes a first holder member and a second holder member. The first holder member and the second holder member are rotatable relative to each other, the discharge port are formed in the first holder member, the scent member may be mounted between the first holder member and the second holder member, and the slider may be disposed between the scent member and the first holder member.

The first holder member: includes a plurality of discharge ports formed so as to constitute a line and a slit connected to one of the discharge ports for guiding the movement of the slider, and the slider: includes the manipulation part inserted through the discharge port connected to the slit such that the manipulation part is moved along the slit.

The first holder member may be further provided with a movement restriction protrusion for limiting a movable range of the slider.

The slider may be provided with an interference protrusion, the holder may include at least one positioning protrusion configured to interfere with the interference protrusion when the slider is moved, and the overlapping area between the discharge port and the opening adjustment port may be maintained by interference between the positioning protrusion and the interference protrusion.

The clothes treatment apparatus may further include a discharge panel defining the treatment chamber, the discharge panel being provided with an air blowing port, through which air blown by the blowing unit is discharged, wherein the scent diffuser may be separably mounted in the discharge panel.

The scent diffuser may be separably mounted in the air blowing port.

The clothes treatment apparatus may further include a steam unit for supplying steam into the treatment chamber, wherein the steam unit may be mounted in the cycle chamber, and the discharge panel may further include a steam discharge port, through which steam generated by the steam unit is discharged.

The embodiments will be described in detail with reference to the following drawings in which like reference numerals refer to like elements wherein:
FIG. 1 is a front perspective view of a clothes treatment apparatus according to a first embodiment of the present invention;
FIG. 2 is a perspective view of a scent diffuser, which is separated from the clothes treatment apparatus shown in FIG. 1;
FIG. 3 is an exploded perspective view of a cycle assembly according to a first embodiment of the present invention;
FIG. 4 is a perspective view of the cycle assembly according to the first embodiment of the present invention;
FIG. 5 is a perspective view of the scent diffuser shown in FIG. 2;
FIG. 6 is a detailed perspective view of part A shown in FIG. 5;
FIG. 7 is a detailed exploded perspective view of part B shown in FIG. 5;
FIG. 8 is an exploded perspective view of the scent diffuser shown in FIG. 5;
FIG. 9 is a perspective view of the scent diffuser shown in FIG. 5 in a state in which the scent diffuser is open;
FIG. 10 is a view showing a first example of the operation of the scent diffuser shown in FIG. 5 when a discharge port of the scent diffuser is completely closed;
FIG. 11 is a sectional view taken along line C-C' of FIG. 10;
FIG. 12 is a view showing a second example of the operation of the scent diffuser shown in FIG. 10 when the discharge port of the scent diffuser is 1/3 open;
FIG. 13 is a sectional view taken along line D-D' of FIG. 12;
FIG. 14 is a view showing a third example of the operation of the scent diffuser shown in FIG. 10 when the discharge port of the scent diffuser is 2/3 open;
FIG. 15 is a sectional view taken along line E-E' of FIG. 14;
FIG. 16 is a view showing a fourth example of the operation of the scent diffuser shown in FIG. 10 when the discharge port of the scent diffuser is completely open;
FIG. 17 is a sectional view taken along line F-F' of FIG. 16; and

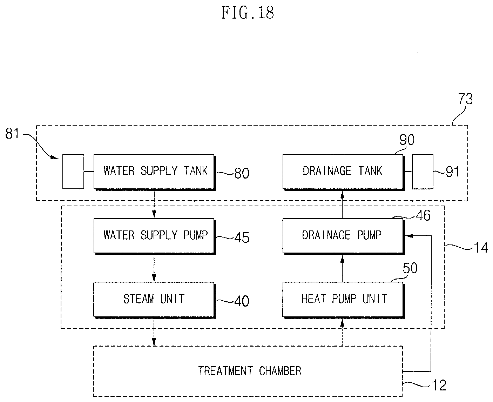

FIG. 18 is a block diagram of the clothes treatment apparatus according to the first embodiment of the present invention.

The present invention will be described in detail with reference to the accompanying drawings.

In the following description of the present invention, a detailed description of known functions or configurations incorporated herein will be omitted when it may make the subject matter of the present invention rather unclear. The same terms may be denoted by different reference numerals if the terms indicate different parts.

The terms used in the following description are terms defined taking into consideration the functions obtained in accordance with the present invention. The definitions of these terms should be determined based on the whole content of this specification because they may be changed in accordance with the intentions of users, such as experimenters and measurers, or usual practices.

In this specification, the terms "first," "second," etc. are used to describe various elements. However, the elements are not limited by the terms. The terms are used only to distinguish one element from another element. For example, a first element may be named a second element, and a second element may be named a first element, without departing from the scope of right of the present invention. It will be understood that the term "and/or" refers to one or more possible combinations of specified relevant items and includes such combinations.

The terms used in this specification are provided only to explain specific embodiments, but are not intended to restrict the present invention. A singular representation may include a plural representation unless it represents a definitely different meaning from the context.

Unless otherwise defined, all terms, including technical and scientific terms, used in this specification have the same meaning as commonly understood by a person having ordinary skill in the art to which the present invention pertains. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In addition, the terms "comprises" and "includes" described herein should be interpreted not to exclude other elements but to further include such other elements since the corresponding elements may be inherent unless mentioned otherwise.

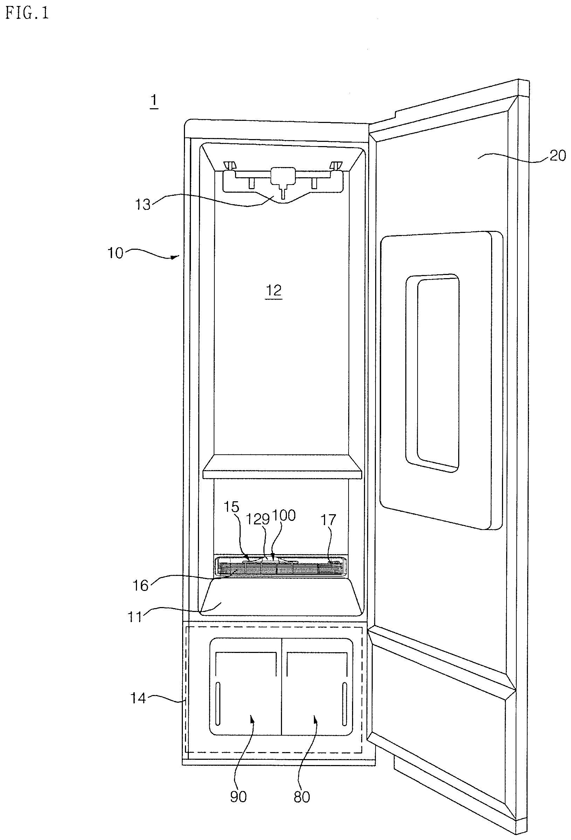

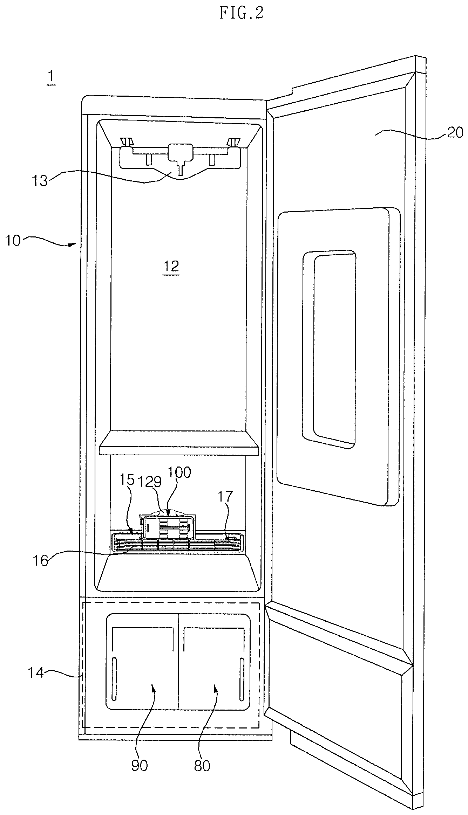

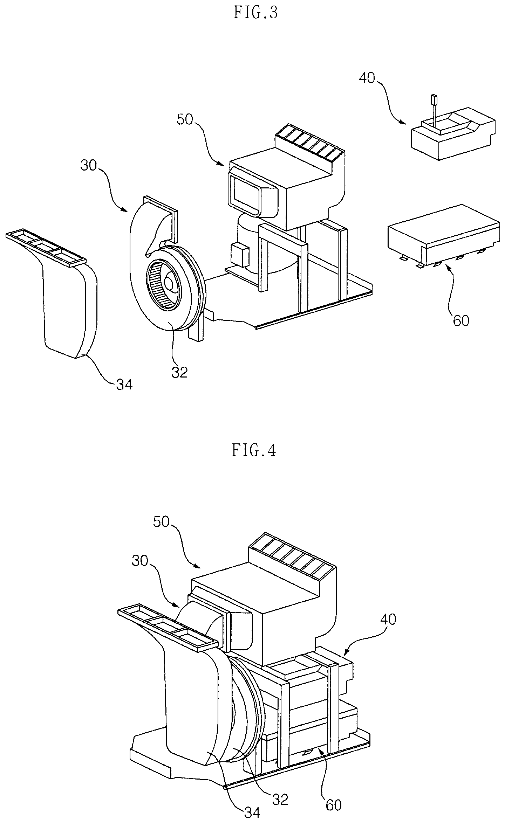

Hereinafter, a clothes treatment apparatus 1 according to a first embodiment of the present invention will be described with reference to FIGS. 1 to 4 and 16.

The clothes treatment apparatus 1 according to this embodiment includes a cabinet 10, which defines a treatment chamber 12 that is open at the front thereof, and a door 20 configured to open and close the front of the cabinet 10.

The interior of the cabinet 10 is partitioned into upper and lower interior parts by a partition plate 11. A treatment chamber 12, in which clothes are hung, is defined in the interior of the cabinet 10 above the partition plate 11. A cycle chamber 14, in which machinery is installed, is defined in the interior of the cabinet 10 below the partition plate 11.

Clothes are hung in the treatment chamber 12. In the treatment chamber 12, wrinkles in the clothes are smoothed, or the clothes are deodorized, by the circulation of steam or air.

A hanger support bar 13 configured to support clothes hangers, on which clothes are hung, is provided in the upper part of the treatment chamber 12. The hanger support bar 13 may be configured to be moved in the treatment chamber 12 in forward and rearward directions, in upward and downward directions, and/or in leftward and rightward directions by a driving device (not shown), such as a motor. The hanger support bar 13 may be periodically reciprocated.

An air blowing port 16 and a steam discharge port 17 are formed in the treatment chamber 12.

In this embodiment, the air blowing port 16 and the steam discharge port 17 are formed in a discharge panel 15.

Unlike this embodiment, the air blowing port 16 and the steam discharge port 17 may be formed in different panels. In this embodiment, the discharge panel 15 constitutes a portion of the cycle chamber 14. The discharge panel 15 is located at the rear side of the partition plate 11. The discharge panel 15 and the partition plate 11 form a continuous surface. The discharge panel 15 is inclined toward the partition plate 11.

Air blown by a blowing unit 30 is discharged through the air blowing port 16.

Steam generated by a steam unit 40 is discharged through the steam discharge port 17.

A blowing unit 30 for circulating air in the treatment chamber 12, a steam unit 40 for supplying steam into the treatment chamber 12, a heat pump unit 50 for conditioning air in the treatment chamber 12, and a control unit 60 for controlling the respective units 30, 40, and 50 are installed in the cycle chamber 14.

In this embodiment, an assembly of machinery, including the blowing unit 30, the steam unit 40, the heat pump unit 50, and the control unit 60, which are required to perform respective cycles of the clothes treatment apparatus, is defined as a cycle assembly.

The blowing unit 30 includes a blowing fan 32 and an inlet duct 34.

The inlet duct 34 is installed at the suction side of the blowing fan 32 to guide air in the treatment chamber 12 to the blowing fan 32.

The blowing fan 32 is rotated to blow air. The blowing fan 32 suctions air from the treatment chamber 12, and discharges the suctioned air to the heat pump unit 50.

When the steam unit 40 is powered on, heat is generated from the steam unit 40. The steam unit 40 converts water supplied from a water supply tank 80, which will be described hereinafter, into steam. The generated steam is discharged into the treatment chamber 12.

In this embodiment, a flow channel is defined such that the steam flows into the treatment chamber 12 via the heat pump unit 50.

The heat pump unit 50 constitutes a heat pump cycle including a compressor, a condenser, an evaporator, and an expansion valve. Based on the operation mode of the heat pump unit 50, cooled air or heated air may be discharged into the treatment chamber 12.

In particular, the heat pump unit 50 may heat air around the condenser through heat exchange with a refrigerant, and may supply the heated air into the treatment chamber 12 through the blowing unit 30. The high-temperature air, which is supplied into the treatment chamber 12, is used to treat clothes that are hung on the clothes hangers, which are supported by the hanger support bar 13. Of course, in case the heat pump unit 50 is not operated, but only the blowing unit is operated, room-temperature air is supplied into the treatment chamber 12. In addition, air cooled by the evaporator may be supplied into the treatment chamber 12 through the blowing unit 30.

The heat pump unit 50 may dehumidify the air in the treatment chamber 12.

A tank module 70 for storing water is installed in front of the cycle chamber 14. The tank module 70 includes a water supply tank 80 for supplying water to the steam unit 40 and a drainage tank 90 for collecting and storing condensed water that is generated in the treatment chamber 12.

A water supply level sensor 81 for sensing the level of water stored in the water supply tank 80 is installed in the water supply tank 80, and a drainage level sensor 91 for sensing the level of water stored in the drainage tank 90 is installed in the drainage tank 90.

Water from the water supply tank 80 flows to the steam unit 40 via a water supply pump 45.

Water that is condensed in the treatment chamber 12 flows to the lower side of the treatment chamber 12 due to gravity, and is then pumped to the drainage tank 90 by a drainage pump 46. Water that is condensed in the heat pump unit 50 also flows to the drainage tank 90 via the drainage pump 46.

The water supply pump 45 or the drainage pump 46 is controlled by the control unit 60.

In this embodiment, a tank module frame 71 is installed in front of the inlet duct 34.

A tank installation space 73 is defined between the tank module frame 71 and the door 20. The tank module frame 71 is coupled to the partition plate 11 to isolate the cycle chamber 14 from the outside.

A tank support bar 75, which interferes with at least one selected from between the water supply tank 80 and the drainage tank 90, is installed in front of the tank installation space 73.

The tank support bar 75 prevents the water supply tank 80 or the drainage tank 90 from being unintentionally separated from the tank installation space 73. The tank support bar 75 supports the front of the water supply tank 80 and the front of the drainage tank 90.

When the door 20 is opened and closed, therefore, the water supply tank 80 and the drainage tank 90 are prevented from being separated from the tank installation space 73.

In this embodiment, the lower end of the water supply tank 80 is placed on the upper end of the tank support bar 75, and the lower end of the drainage tank 90 is placed on the upper end of the tank support bar 75.

A tank support end 79, which interferes with the tank support bar 75, is formed on at least one selected from between the water supply tank 80 and the drainage tank 90.

The tank support end 79 is concavely recessed.

The front of the tank support bar 75 and the front of the water supply tank 80 may form a continuous surface due to the tank support end 79. In addition, the front of the tank support bar 75 and the front of the drainage tank 90 may form a continuous surface due to the tank support end 79.

The water supply tank 80 and the drainage tank 90 are disposed in the tank installation space 73 such that the water supply tank 80 and the drainage tank 90 are arranged parallel to each other in rightward and leftward directions.

When the door 20 is opened, the water supply tank 80 and the drainage tank 90 are exposed to a user.

The water supply tank 80 and the drainage tank 90 may be withdrawn by the user.

The water supply tank 80 and the drainage tank 90 may be separated from the tank module frame 71. The water supply tank 80 and the drainage tank 90 may be separably mounted in the tank installation space 73.

The water supply tank 80 is connected to the steam unit 40 to supply water to the steam unit 40. The drainage tank 90 is connected to the treatment chamber 12 to store water discharged from the treatment chamber 12 or the heat pump unit 50.

The drainage tank 90 is identical in function to the water supply tank 80. The drainage tank 90 is disposed alongside the water supply tank 80.

The clothes treatment apparatus 1 may further include a scent diffuser 100 configured to diffuse scent into the treatment chamber 12. The scent diffuser 100 may be disposed in the treatment chamber 12. After the scent diffuser 100 is completely used, the user may replace the scent diffuser 100 with a new one.

In this embodiment, the scent diffuser 100 is separably mounted in the discharge panel 15. The scent diffuser 100 is inserted into an opening formed in the discharge panel 15. The user may simply pull the scent diffuser 100 to separate the scent diffuser 100 from the discharge panel 15.

The scent diffuser 100 may be provided with a handle 129, which is exposed through the opening. The user may pull the scent diffuser 100 while holding the handle 129 to separate the scent diffuser 100 from the discharge panel 15.

The scent diffuser 100 may be provided in a flow channel along which air blown by the blowing unit 30 is guided to the air blowing port 16. Alternatively, the scent diffuser 100 may be separably provided at any constructional component, such as the door 20, other than the discharge panel 15 as long as the scent diffuser 100 can supply scent into the treatment chamber 12.

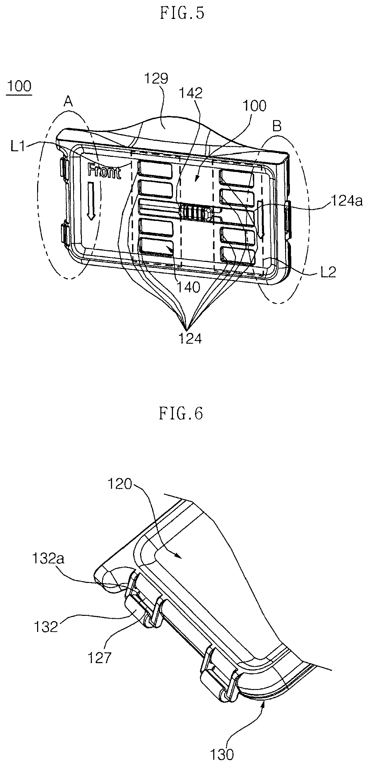

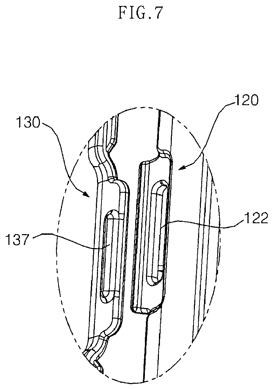

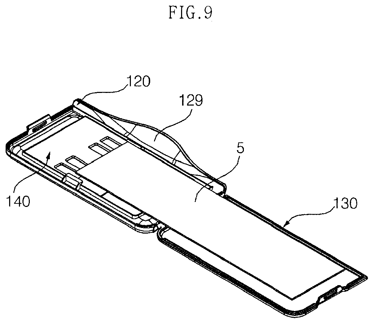

Hereinafter, a scent diffuser 100 according to a first embodiment of the present invention will be described in detail with reference to FIGS. 5 to 17.

A replaceable scent member 5 is mounted in the scent diffuser 100.

The scent diffuser 100 is separably mounted in the discharge panel 15.

The scent diffuser 100 includes a holder 110, in which the scent member 5 is mounted, the holder 110 having at least one discharge port 124, and a slider 140 movably provided at the holder 110 for adjusting the extent of opening of the discharge port 124 based on the position thereof.

The scent member 5 is made of a material that diffuses scent. When the scent member 5 is exposed to the air, the scent member 5 diffuses scent into the air. In this embodiment, the scent member 5 is provided in a sheet type form. Unlike this embodiment, the scent member 5 may be provided in various other forms, such as a particle type form and a liquid type form.

When the scent member 5 is completely used, the user may separate the scent member 5 from the discharge panel 15 of the treatment chamber 12, and may then mount a new one in the discharge panel 15 of the treatment chamber 12.

The holder 110 is provided with a scent member installation space 111, in which the scent member 5 is received. Scent discharged from the scent member 5 may be diffused to the outside through the discharge port 124.

A plurality of discharge ports 124 may be provided. In this embodiment, the discharge ports 124 constitute a plurality of lines. The discharge ports 124 constitute a plurality of lines in a longitudinal direction.

The discharge ports 124 constitute two lines L1 and L2 (see FIG. 5), which are spaced apart from each other in a direction in which the slider 140 moves. The extents of opening of the discharge ports 124 constituting each line L1 or L2 are simultaneously adjusted by the slider 140.

Hereinafter, the discharge ports 124 constituting the first line L1 will be referred to as first discharge ports, and the discharge ports 124 constituting the second line L2 will be referred to as second discharge ports, as needed.

At least a portion of the slider 140 is exposed outward from the holder 110 such that the user can manipulate the slider 140. The slider 140 may be moved by a user's manipulation, whereby the extent of opening of the first discharge ports and the second discharge ports may be adjusted.

The holder 110 includes a first holder member 120 and a second holder member 130. The scent member installation space 111, in which the scent member 5 is mounted, is formed between the first holder member 120 and the second holder member 130.

The scent member installation space 111 may be formed in the first holder member 120 or the second holder member 130. In this embodiment, the scent member installation space 111 is formed in the second holder member 130. A slider installation space 113, in which the slider 140 is mounted, is formed in the first holder member 120.

The discharge ports 124 may be formed in the first holder member 120 or the second holder member 130. In this embodiment, the discharge ports 124 are formed in the first holder member 120.

The first holder member 120 includes a first housing 121, in which the slider installation space 113, in which the slider 140 is movably received, is formed.

The second holder member 130 includes a second housing 131, in which the scent member installation space 111, in which the scent member 5 is received, is formed.

The scent member 5 and the slider 140 are arranged opposite to each other.

The discharge ports 124 and a slit 125 are formed in the first housing 121.

The slit 125 extends in a longitudinal direction of the first holder member 120 to guide the movement of the slider 140.

The slit 125 may be formed separately from the discharge ports 124. In this embodiment, the slit 125 is connected to one of the discharge ports 124, i.e. a discharge port 124a.

In this embodiment, the discharge port 124a connected to the slit 125 is used as a manipulation part installation hole, in which the slider 140 is mounted. Unlike this embodiment, the manipulation part installation hole may be formed separately from the discharge port 124a.

The discharge port 124a is formed so as to be wider than the slit 125.

The first holder member 120 and the second holder member 130 may be separably coupled to each other. Furthermore, the first holder member 120 and the second holder member 130 may be connected to each other such that the first holder member 120 and the second holder member 130 can be rotated relative to each other.

To this end, one selected from between the first holder member 120 and the second holder member 130 may include a pivot 127, which is used as a rotational shaft about which the other selected from between the first holder member 120 and the second holder member 130 can be rotated, and the other selected from between the first holder member 120 and the second holder member 130 may include a hinge coupling unit 132, which is rotatably coupled to the pivot 127.

The hinge coupling unit 132 may be separably coupled to the pivot 127. The hinge coupling unit 132 extends so as to correspond to the pivot 127.

The hinge coupling unit 132 may be provided with a recess 132a (see FIG. 6), through which the pivot 127 is inserted. The hinge coupling unit 132 is made of an elastic material (e.g. a synthetic resin) such that the circumference of the recess 132a is elastically deformed to some extent when the pivot 127 is inserted into the recess 132a. The first holder member 120, the second holder member 130, and/or the slider 140 may be made of a synthetic resin by injection molding.

The pivot 127 is forcibly inserted into the hinge coupling unit 132. When the pivot 127 is forcibly inserted into the hinge coupling unit 132, the hinge coupling unit 132 is elastically deformed such that the pivot 127 can be inserted into the hinge coupling unit 132. When the pivot 127 is separated from the hinge coupling unit 132, the hinge coupling unit 132 may also be elastically deformed.

The slider 140 includes a plate 141 disposed in the holder 110 such that the plate 141 can move along the holder 110, a manipulation part 142 formed at the plate 141, the manipulation part 142 being exposed outward from the holder 110, the manipulation part 142 being manipulated by a user, the manipulation part 142 being configured to move along the slit 125, and opening adjustment ports 144 formed in the plate 141, the opening adjustment ports 144 being configured to overlap the discharge ports 124 so as to expose the scent member 5.

The plate 141 may be disposed in the holder 110 to change the area that covers the discharge ports 124 based on the position of the slider 140.

The manipulation part 142 is the exposed portion of the slider 140, which is exposed outward from the holder 110. The manipulation part 142 constitutes a portion of the slider 140. In this embodiment, the manipulation part 142 protrudes from the plate 141. Unlike this embodiment, in a case in which the holder is configured to have a structure in which a portion of the plate 141 is exposed outward, the exposed portion of the plate 141, which is exposed outward from the holder, may be used as the manipulation part.

The extent of opening of the discharge ports 124 is adjusted based on the area covered by the plate 141. One side part of the plate 141 adjusts the extent of opening of the first discharge ports 124, and the other side part of the plate 141 adjusts the extent of opening of the second discharge ports 124, on the basis of the manipulation part 142.

The slit 125, which extends in a direction in which the manipulation part 142 moves, may be formed in the holder 110. In this case, the manipulation part 142 may move along the slit 125. The slit 125 may extend from one of the discharge ports 124, i.e. the discharge port 124a. The discharge port 124a, which is connected to the slit 125, is sufficiently large to allow the manipulation part 142 to pass therethrough.

The manipulation part 142 is inserted into the discharge port 124a from the inside of the discharge port 124a such that the manipulation part 142 protrudes outward from the first holder member 120.

The slit 125 is formed so as to be narrower than each of the discharge ports 124. The slit 125 extends in a direction in which the slider 140 moves. The manipulation part 142 is connected to the plate 141 via a connection part (not shown) that is narrow enough to pass through the slit 125.

The amount of scent discharged from the scent diffuser 100 may be set based on the extent of opening of the discharge ports 124. The extent of opening of the discharge ports 124 corresponds to the position of the manipulation part 142.

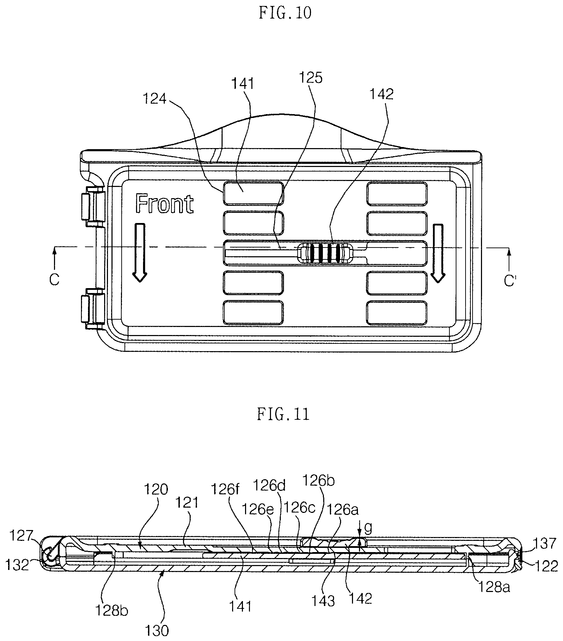

The plate 141 moves to adjust the area of the discharge ports 124 that is covered. Referring to FIGS. 10 to 17, it can be seen that the extent of opening of the discharge ports 124 can be adjusted through several stages based on the position of the manipulation part 142.

In the slider 140, the manipulation part 142 is located at the middle of the opening adjustment ports 144. The opening adjustment ports 144 disposed at the upper side of the manipulation part 142 and the opening adjustment ports 144 disposed at the lower side of the manipulation part 142 are arranged symmetrically.

The slit 125 is located in the middle of the holder 110. The manipulation part 142 moves along the slit 125, which is located in the middle of the holder 110. The user may intuitively check the position of the manipulation part 142.

A plurality of opening adjustment ports 144 is provided. The opening adjustment ports 144 simultaneously overlap the discharge ports 124 constituting the first line L1 or the discharge ports 124 constituting the second line L2.

For example, the opening adjustment ports 144 may simultaneously adjust the extent of opening of the discharge ports 124 constituting the first line L1. The scent member 5 is exposed from the discharge ports 124 through the opening adjustment ports 144.

In this embodiment, one opening adjustment port 144 overlaps one discharge port 124.

The opening adjustment ports 144 and the manipulation part 142 are arranged in a line.

The opening adjustment ports 144 move the same distance as the manipulation part 142.

In the above arrangement, it is possible for the user to intuitively check the extent of opening of the discharge ports 124. The extent of opening of the discharge ports 124 is adjusted based on the position of the manipulation part 142.

When the manipulation part 142 is completely aligned with the discharge ports 124, the discharge ports 124 are completely open. On the other hand, when the manipulation part 142 completely deviates from the discharge ports 124, the discharge ports 124 are completely closed.

The slider 140 may be provided with at least one interference protrusion 143.

The holder 110 may be provided with one or more positioning protrusions 126a to 126f, which are located in a path along which the interference protrusion 143 moves and which interfere with the interference protrusion 143 when the slider 140 is moved.

The number of positioning protrusions 126a to 126f is greater than the number of interference protrusions 143. When the user moves the manipulation part 142, the user may perceive the sensation of the interference protrusions 143 moving over the positioning protrusions 126a to 126f.

Since the positions of the positioning protrusions 126a to 126f are set so as to correspond to the extent of opening of the discharge ports 124, the user may manipulate the slider 140 such that the slider 140 can be appropriately positioned based on a predetermined extent of opening of the discharge ports 124.

The holder 110 may be provided with at least one movement restriction protrusion 128a and 128b for limiting a movable range of the slider 140. The slider 140 may be disposed between the movement restriction protrusion 128a and 128b.

The movement of the slider 140 in one direction is restricted by the first movement restriction protrusion 128a, and the movement of the slider 140 in the other direction is restricted by the second movement restriction protrusion 128b.

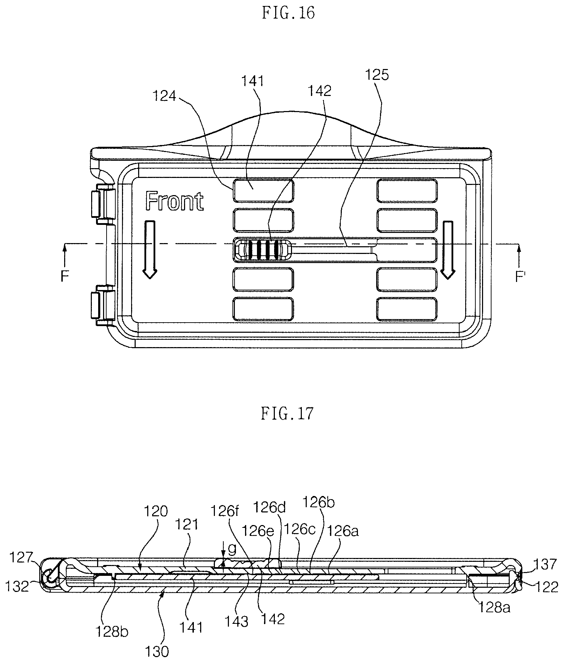

The discharge ports 124 may be completely closed (see FIG. 10 or 11) at a position at which the slider 140 cannot move any further due to the first movement restriction protrusion 128a. The discharge ports 124 may be completely open (see FIG. 16 or 17) at a position at which the slider 140 cannot move any further due to the second movement restriction protrusion 128b.

The movement restriction protrusions 128a and 128b are formed in a direction crossing the movement path of the slider 140. In this embodiment, each of the movement restriction protrusions 128a and 128b is formed to have a rib shape.

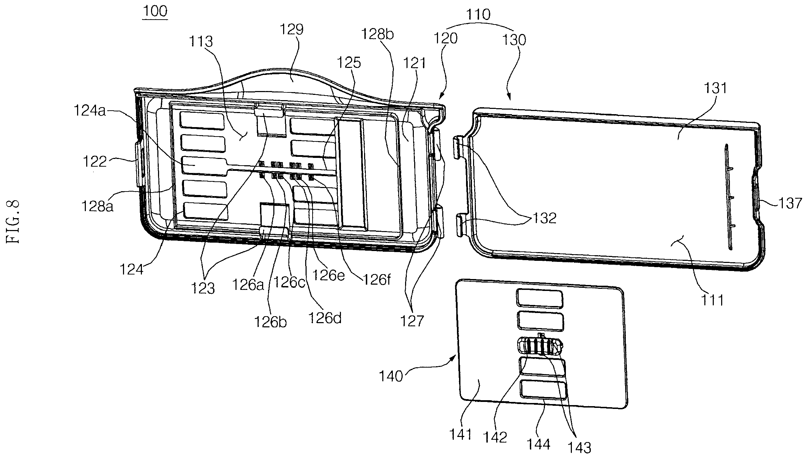

Referring to FIG. 8, the interference protrusions 143 may be provided at opposite sides of the manipulation part 142.

In the first housing 121, positioning protrusions 126a to 126f for restricting the position of one interference protrusion 143 may be disposed at one side of the slit 125 such that the positioning protrusions 126a to 126f are arranged in a line.

In the first housing 121, positioning protrusions 126a to 126f for restricting the position of the other interference protrusion 143 may be disposed at the other side of the slit 125 such that the positioning protrusions 126a to 126f are arranged in a line. The positioning protrusions 126a to 126f for restricting the position of one interference protrusion 143 and the positioning protrusions 126a to 126f for restricting the position of the other interference protrusion 143 are disposed symmetrically.

Referring to FIGS. 10 to 17, the scent diffuser 100 according to this embodiment is configured such that the discharge ports 124 can be adjusted to have four extents of opening, for example fully open, 1/3 open, 2/3 open, and fully closed. To this end, the six positioning protrusions 126a to 126f are arranged along the movement path of the interference protrusions 143 in a line.

Referring to FIG. 10 or 11, the first positioning protrusion 126a finally interferes with the interference protrusion 143 when the slider 140 is moved from the left to the right of the figure. After the interference protrusion 143 moves over the first positioning protrusion 126a, the slider 140 comes into contact with the first positioning protrusion 126a, with the result that the slider 140 does not move any further.

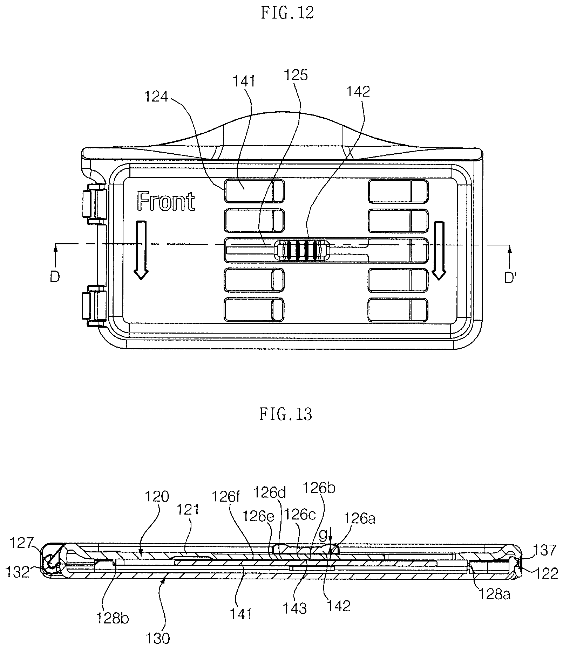

Referring to FIG. 12 or 13, the second positioning protrusion 126b and the third positioning protrusion 126c are provided so as to set the position of the slider 140 at which the discharge ports 124 are 1/3 open.

In a state in which the discharge ports 124 are 1/3 open, the interference protrusion 143 is located between the second positioning protrusion 126b and the third positioning protrusion 126c (hereinafter, referred to as a 1/3 open position).

For example, when the slider 140 is moved from the position at which the discharge ports 124 are completely closed, the user may perceive the sensation of the interference protrusion 143 moving over the second positioning protrusion 126b, and may then immediately perceive the sensation of the interference protrusion 143 moving over the third positioning protrusion 126c. As a result, the user may not move the slider 140 any further, whereby the slider 140 is located at the 1/3 open position.

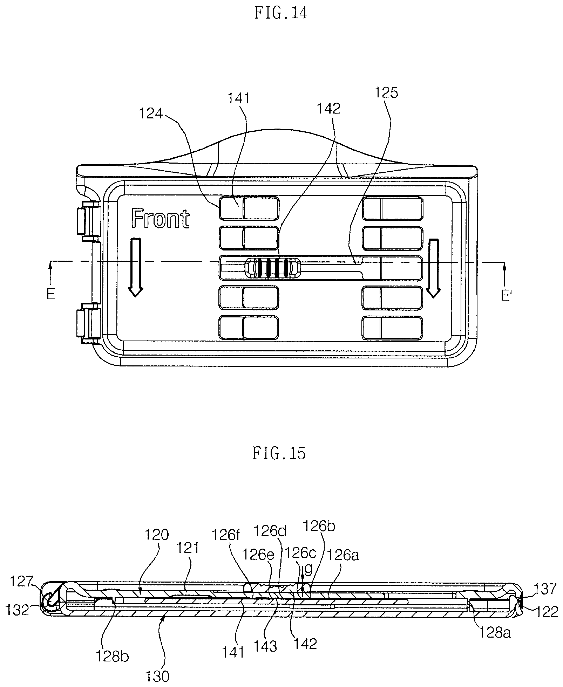

Referring to FIG. 14 or 15, the fourth positioning protrusion 126d and the fifth positioning protrusion 126e are provided so as to correspond to the position of the slider 140 at which the discharge ports 124 are 2/3 open.

In a state in which the discharge ports 124 are 2/3 open, the interference protrusion 143 is located between the fourth positioning protrusion 126d and the fifth positioning protrusion 126e (hereinafter, referred to as a 2/3 open position).

For example, when the slider 140 is moved from the position at which the discharge ports 124 are 1/3 open, the user may perceive the sensation of the interference protrusion 143 moving over the fourth positioning protrusion 126d, and may then immediately perceive the sensation of the interference protrusion 143 moving over the fifth positioning protrusion 126e. As a result, the user may not move the slider 140 any further, whereby the slider 140 is located at the 2/3 open position.

Referring to FIG. 16 or 17, when the slider 140 is moved from the position at which the discharge ports 124 are 2/3 open, the interference protrusion 143 moves over the six positioning protrusion 126f. At this time, the slider 140 comes into contact with the second movement restriction protrusion 128b, with the result that the slider 140 does not move any further.

Consequently, when the user perceives the sensation of the interference protrusions 143 moving over the positioning protrusions 126a to 126f, and then perceives the sensation of the slider 140 contacting the second movement restriction protrusion 128b, the user may recognize that the slider 140 has reached the position at which the slider 140 cannot move any further, i.e. the position at which the discharge ports 124 are completely open.

As described above, the positioning protrusions 126a to 126f may enable the user to recognize the positions of the slider 140 that correspond to the extent of opening of the discharge ports 124 based on the sensation of manipulation during the manipulation of the slider 140, and may prevent the movement of the slider 140 after the interference protrusions 143 are located at the predetermined positions (e.g. the 1/3 open position and the 2/3 open position).

According to embodiments, the positioning protrusions may be configured differently. For example, a larger number of positioning protrusions may be provided in order to adjust the extent of opening of the discharge ports 124 through a larger number of stages.

In another embodiment, one selected from between the slider 140 and the holder 110 may include an interference protrusion, and the other selected from between the slider 140 and the holder 110 may include positioning insertion parts, into which the interference protrusion is inserted when the slider 140 is located at predetermined positions. In this case, the positioning insertion parts may be provided at positions corresponding to the extent of opening of the discharge ports 124 in a manner similar to the positioning protrusions 126a to 126f in the previous embodiment, and the interference protrusion may be inserted into the positioning insertion parts during the movement of the slider 140, whereby the user may perceive the sensation of manipulation.

As is apparent from the above description, the scent diffuser and the clothes treatment apparatus according to the present invention have the following effects.

First, it is possible for the user to arbitrarily adjust the amount of scent to be discharged through the manipulation part.

Second, it is possible for the user to adjust the amount of scent to be discharged based on simple manipulation performed by the user without using additional electric power.

Third, it is possible for the user to separate the scent diffuser from the treatment chamber and to replace a completely used scent member with a new one.

Fourth, the manipulation part is aligned with the opening adjustment ports. Consequently, it is possible for the user to intuitively check the extent of opening of the discharge ports.

Fifth, the manipulation part is mounted through the discharge port that is connected to the slit. Consequently, it is possible to omit an additional structure for installing the manipulation part.

Sixth, it is possible to maintain the position to which the slider has been moved based on interference between the interference protrusions and the positioning protrusions, whereby it is possible to maintain the overlapping area between the discharge ports and the opening adjustment ports.

Seventh, the scent member is covered by the slider. Consequently, it is possible to supply scent into the treatment chamber as need, whereby it is possible to extend the lifespan of the scent member.

## Claims

1. A clothes treatment apparatus comprising:
a cabinet (10) having a treatment chamber (12) for allowing clothes to be hung therein and a cycle chamber (14) for allowing machinery to be installed therein;
a blowing unit (30) installed in the cycle chamber for circulating air in the treatment chamber; and
a scent diffuser (100) separably disposed in the treatment chamber for diffusing scent into the treatment chamber,
wherein the scent diffuser comprises:
a holder (110), in which a replaceable scent member (5) is mounted, the holder having at least one discharge port (124), through which scent emitted from the scent member is discharged; and
a slider (140) having a manipulation part (142) exposed outward from the holder, the slider being movable relative to the holder when force generated by manipulation of the manipulation part is transmitted to the slider, the slider being configured to adjust an extent of opening of the discharge port based on a position to which the slider has been moved,
**characterised in that** the holder (110) comprises a first holder member (120) and a second holder member (130), the first holder member and the second holder member being rotatable relative to each other,
a scent member installation space (111), in which the scent member (5) is mounted, and a slider installation space (113), in which the slider (140) is mounted, are formed between the first holder member and the second holder member,
the first holder member comprises: a plurality of discharge ports (124) formed so as to constitute a line; and a slit (125) connected to one of the discharge ports for guiding movement of the slider, and
the slider (140) comprises: a plate (141) disposed in the slider installation space such that the plate moves along the holder so as to cover the discharge ports; an opening adjustment port (144) formed in the plate, the opening adjustment port being configured to overlap the discharge port so as to expose the scent member outward; and the manipulation part (142) inserted through the discharge port connected to the slit such that the manipulation part is exposed outward from the first holder member, the manipulation part being moved along the slit according to a user's manipulation of the manipulation part.

2. The clothes treatment apparatus according to claim 1, further comprising:
a discharge panel (15) defining the treatment chamber, the discharge panel being provided with an air blowing port (16), through which air blown by the blowing unit is discharged, wherein
the scent diffuser (100) is separably mounted in the discharge panel.

3. The clothes treatment apparatus according to claim 2, wherein the scent diffuser (100) is separably mounted in the air blowing port (16).

4. The clothes treatment apparatus according to claim 2 or 3, further comprising:
a steam unit (40) for supplying steam into the treatment chamber (12), wherein
the steam unit is mounted in the cycle chamber (14), and
the discharge panel (15) further comprises a steam discharge port (17), through which steam generated by the steam unit is discharged.

5. The clothes treatment apparatus according to claim 1, wherein
the plate (141) is disposed in the holder such that the plate is moved simultaneously with the manipulation part so as to change an area that covers the discharge port.

6. The clothes treatment apparatus according to claim 1, wherein the opening adjustment port (144) comprises a plurality of opening adjustment ports, and the opening adjustment ports and the manipulation part (142) are arranged in a line.

7. The clothes treatment apparatus according to claim 1, wherein
the slider (140) is provided with an interference protrusion (143),
the holder (110) comprises at least one positioning protrusion (126a to 126f) configured to interfere with the interference protrusion when the slider is moved, and
an overlapping area between the discharge port (124) and the opening adjustment port (144) is maintained by interference between the positioning protrusion and the interference protrusion.

8. The clothes treatment apparatus according to claim 1, wherein
the slider (140) is disposed between the scent member and the first holder member.

9. The clothes treatment apparatus according to claim 1, wherein
the slider installation space (113) is formed in the first holder member; and
the scent member installation space (111) is formed in the second holder member.

## Patentansprüche

1. Wäschebehandlungsvorrichtung, die aufweist:
einen Kasten (10) mit einer Behandlungskammer (12), um zuzulassen, dass Wäsche darin aufgehängt wird, und eine Zirkulationskammer (14), die zulässt, dass Maschinenanlagen darin installiert werden;
eine Gebläseeinheit (30), die in der Zirkulationskammer installiert ist, um Luft in der Behandlungskammer zu zirkulieren; und
einen Duftdiffusor (100), der getrennt in der Behandlungskammer angeordnet ist, um einen Duft in die Behandlungskammer zu diffundieren, wobei der Duftdiffusor aufweist:
eine Halterung (110), in der ein austauschbares Duftelement (5) montiert ist, wobei die Halterung wenigstens eine Abgabemündung (124) hat, durch die von dem Duftelement abgesonderter Duft abgegeben wird; und
einen Schieber (140) mit einem Handhabungsteil (142), der von der Halterung nach außen freiliegt, wobei der Schieber relativ zu der Halterung beweglich ist, wenn eine durch die Handhabung des Handhabungsteils erzeugte Kraft auf den Schieber übertragen wird, wobei der Schieber aufgebaut ist, um ein Maß der Öffnung der Abgabemündung basierend auf einer Position, zu der der Schieber bewegt wurde, einzustellen,
**dadurch gekennzeichnet, dass**
die Halterung (110) ein erstes Halterungselement (120) und ein zweites Halterungselement (130) aufweist, wobei das erste Halterungselement und das zweite Halterungselement relativ zueinander drehbar sind,
ein Duftelement-Installationsraum (111), in dem das Duftelement (5) montiert ist, und ein Schieberinstallationsraum (113), in dem der Schieber (140) montiert ist, zwischen dem ersten Halterungselement und dem zweiten Halterungselement ausgebildet sind,
das erste Halterungselement aufweist: mehrere Abgabemündungen (124), die derart ausgebildet sind, dass sie eine Linie bilden; und einen Schlitz (125), der mit einer der Abgabemündungen verbunden ist, um die Bewegung des Schiebers zu führen, und
der Schieber (140) aufweist: eine Platte (141), die in dem Schieberinstallationsraum derart angeordnet ist, dass die Platte sich entlang der Halterung bewegt, um die Abgabemündungen zu bedecken; eine Öffnungseinstellungsmündung (144), die in der Platte ausgebildet ist, wobei die Öffnungseinstellungsmündung aufgebaut ist, um die Abgabemündung zu überlappen, um das Duftelement nach außen freilzulegen; und
der Handhabungsteil (142), der durch die Abgabemündung eingesetzt ist, derart mit dem Schlitz verbunden ist, dass der Handhabungsteil von dem ersten Halterungselement nach außen freiliegt, wobei der Handhabungsteil gemäß einer Handhabung des Handhabungsteils durch einen Benutzer entlang des Schlitzes bewegt wird.

2. Wäschebehandlungsvorrichtung nach Anspruch 1, die ferner aufweist:
eine Abgabeplatte (15), die die Behandlungskammer begrenzt, wobei die Abgabeplatte mit einer Luftblasmündung (16) versehen ist, durch die von der Gebläseeinheit geblasene Luft abgegeben wird, wobei der Duftdiffusor (100) trennbar in der Abgabeplatte montiert ist.

3. Wäschebehandlungsvorrichtung nach Anspruch 2, wobei der Duftdiffusor (100) trennbar in der Luftblasmündung (16) montiert ist.

4. Wäschebehandlungsvorrichtung nach Anspruch 2 oder 3, die ferner aufweist:
eine Dampfeinheit (40) zum Zuführen von Dampf in die Behandlungskammer (12), wobei
die Dampfeinheit in der Zirkulationskammer (14) montiert ist, und
die Abgabeplatte (15) ferner eine Dampfabgabemündung (17) aufweist, durch die von der Dampfeinheit erzeugter Dampf abgegeben wird.

5. Wäschebehandlungsvorrichtung nach Anspruch 1, wobei
die Platte (141) derart in der Halterung angeordnet ist, dass die Platte gleichzeitig mit dem Handhabungsteil bewegt wird, um eine Fläche, die die Abgabemündung bedeckt, zu ändern.

6. Wäschebehandlungsvorrichtung nach Anspruch 1, wobei die Öffnungseinstellungsmündung (144) mehrere Öffnungseinstellungsmündungen aufweist, und die Öffnungseinstellungsmündungen des Handhabungsteils (142) in einer Linie angeordnet sind.

7. Wäschebehandlungsvorrichtung nach Anspruch 1, wobei
der Schieber (140) mit einem Eingreifvorsprung (143) versehen ist,
die Halterung (110) wenigstens einen Positionierungsvorsprung (126a bis 126f) aufweist, der aufgebaut ist, um mit dem Eingreifvorsprung einzugreifen, wenn der Schieber bewegt wird, und
eine Überlappungsfläche zwischen der Abgabemündung (124) und der Öffnungseinstellungsmündung (144) durch den Eingriff zwischen dem Positionierungsvorsprung und dem Eingreifvorsprung aufrecht erhalten wird.

8. Wäschebehandlungsvorrichtung nach Anspruch 1, wobei
der Schieber (140) zwischen dem Duftelement und dem ersten Halterungselement angeordnet ist.

9. Wäschebehandlungsvorrichtung nach Anspruch 1, wobei
der Schieberinstallationsraum (113) in dem ersten Halterungselement ausgebildet ist; und
der Duftelement-Installationsraum (111) in dem zweiten Halterungselement ausgebildet ist.

## Revendications

1. Appareil de traitement de linge comprenant :
une armoire (10) ayant une chambre de traitement (12) pour permettre au linge d'être accroché à l'intérieur de celle-ci et une chambre de cycle (14) pour permettre à la machinerie d'être installée à l'intérieur de celle-ci ;
une unité de soufflage (30) installée dans la chambre de cycle pour la circulation d'air dans la chambre de traitement ; et
un diffuseur de parfum (100) disposé séparablement dans la chambre de traitement pour la diffusion de parfum dans la chambre de traitement, dans lequel le diffuseur de parfum comprend :
un support (110), dans lequel un élément de parfum remplaçable (5) est monté, le support ayant au moins un orifice d'évacuation (124), à travers lequel du parfum émis par l'élément de parfum est évacué ; et
un coulisseau (140) ayant une partie de manipulation (142) exposée vers l'extérieur du support, le coulisseau étant mobile par rapport au support lorsqu'une force générée par manipulation de la partie de manipulation est transmise au coulisseau, le coulisseau étant configuré pour ajuster une étendue d'ouverture de l'orifice d'évacuation sur la base d'une position vers laquelle le coulisseau a été déplacé,
**caractérisé en ce que**
le support (110) comprend
un premier élément de support (120) et un deuxième élément de support (130), le premier élément de support et le deuxième élément de support étant rotatifs l'un par rapport à l'autre,
un espace d'installation d'élément de parfum (111), dans lequel l'élément de parfum (5) est monté, et un espace d'installation de coulisseau (113), dans lequel le coulisseau (140) est monté, sont formés entre le premier élément de support et le deuxième élément de support,
le premier élément de support comprend : une pluralité d'orifices d'évacuation (124) formée de façon à constituer une ligne ; et une fente (125) reliée à l'un des orifices d'évacuation pour guider le mouvement du coulisseau, et
le coulisseau (140) comprend : une plaque (141) disposée dans l'espace d'installation de coulisseau de sorte que la plaque se déplace le long du support de façon à couvrir les orifices d'évacuation ; un orifice d'ajustement d'ouverture (144) formé dans la plaque, l'orifice d'ajustement d'ouverture étant configuré pour chevaucher l'orifice d'évacuation de façon à exposer l'élément de parfum vers l'extérieur ; et la partie de manipulation (142) insérée à travers l'orifice d'évacuation relié à la fente de sorte que la partie de manipulation est exposée vers l'extérieur du premier élément de support, la partie de manipulation étant déplacée le long de la fente en fonction d'une manipulation de l'utilisateur de la partie de manipulation.

2. Appareil de traitement de linge selon la revendication 1, comprenant en outre :
un panneau d'évacuation (15) définissant la chambre de traitement, le panneau d'évacuation étant doté d'un orifice de soufflage d'air (16), à travers lequel de l'air soufflé par l'unité de soufflage est évacué, dans lequel
le diffuseur de parfum (100) est monté séparablement dans le panneau d'évacuation.

3. Appareil de traitement de linge selon la revendication 2, dans lequel le diffuseur de parfum (100) est monté séparablement dans l'orifice de soufflage d'air (16).

4. Appareil de traitement de linge selon la revendication 2 ou 3, comprenant en outre :
une unité de vapeur (40) pour la fourniture de vapeur dans la chambre de traitement (12), dans lequel
l'unité de vapeur est montée dans la chambre de cycle (14), et
le panneau d'évacuation (15) comprend en outre un orifice d'évacuation de vapeur (17), à travers lequel de la vapeur générée par l'unité de vapeur est évacuée.

5. Appareil de traitement de linge selon la revendication 1, dans lequel la plaque (141) est disposée dans le support de sorte que la plaque est déplacée simultanément avec la partie de manipulation de façon à changer une zone qui couvre l'orifice d'évacuation.

6. Appareil de traitement de linge selon la revendication 1, dans lequel l'orifice d'ajustement d'ouverture (144) comprend une pluralité d'orifices d'ajustement d'ouverture, et les orifices d'ajustement d'ouverture et la partie de manipulation (142) sont disposés en ligne.

7. Appareil de traitement de linge selon la revendication 1, dans lequel
le coulisseau (140) est doté d'une saillie d'interférence (143),
le support (110) comprend au moins une saillie de positionnement (126a à 126f) configurée pour interférer avec la saillie d'interférence lorsque le coulisseau est déplacé, et
une zone de chevauchement entre l'orifice d'évacuation (124) et l'orifice d'ajustement d'ouverture (144) est maintenue par interférence entre la saillie de positionnement et la saillie d'interférence.

8. Appareil de traitement de linge selon la revendication 1, dans lequel
le coulisseau (140) est disposé entre l'élément de parfum et le premier élément de support.

9. Appareil de traitement de linge selon la revendication 1, dans lequel
l'espace d'installation de coulisseau (113) est formé dans le premier élément de support ; et
l'espace d'installation d'élément de parfum (111) est formé dans le deuxième élément de support.
